**Europäisches Patentamt**

**European Patent Office**

**Office européen des brevets**

(19)

(11) Veröffentlichungsnummer: **0 362 663**

**A1**

(12)

# EUROPÄISCHE PATENTANMELDUNG

(21) Anmeldenummer: **89117700.8**

(22) Anmeldetag: **25.09.89**

(51) Int. Cl.⁵: **A61K 7/09**

(30) Priorität: **04.10.88 DE 3833681**

(43) Veröffentlichungstag der Anmeldung:
**11.04.90 Patentblatt 90/15**

(84) Benannte Vertragsstaaten:
**ES GR**

(71) Anmelder: **Henkel Kommanditgesellschaft auf Aktien**
**Henkelstrasse 67**
**D-4000 Düsseldorf 13(DE)**

(72) Erfinder: **Klenk, Rudolf, Dr.**
**An den Pappeln 14**
**D-4048 Grevenbroich 5(DE)**
Erfinder: **Hollenberg, Detlef, Dr.**
**Fliederweg 31**
**D-4006 Erkrath 2(DE)**

(54) **Dauerwellmittel.**

(57) Die Erfindung betrifft wäßrige Mittel zur Durchführung der reduzierenden Stufe des Dauerwellverfahrens für menschliche Haare, die als Reduktionsmittel Cysteamin, Cystein und/oder ein Derivat des Cysteins mit freier SH-Funktion in Kombination mit mindestens einer weiteren Mercapto-Verbindung, ausgewählt aus Mercaptoethanol, Thioäpfelsäure, Thiomilchsäure und α-Mercaptoethansulfonsäure enthalten.

EP 0 362 663 A1

## Dauerwellmittel

Die Erfindung betrifft wäßrige Mittel zur Durchführung der reduzierenden Stufe eines Dauerwellverfahrens für menschliche Haare.

Das Einbringen von Krausen und Wellen in das Haar und die Verstärkung einer bereits vorhandenen natürlichen Haarwellung sind wichtige Verfahren der dekorativen Kosmetik. Um eine dauerhafte, d. h. den üblichen atmosphärischen Einwirkungen widerstehende, Wellung zu erhalten, wird die Verformung mittels einer reversiblen Spaltung von im Haar vorhandenen Disulfid-Brücken durchgeführt. Dabei wird das Haar in einem ersten Schritt mit einer Reduktionsmittel-haltigen Zubereitung behandelt. Durch die so erzielte Spaltung von im Haar vorhandenen Disulfidbrücken werden die Haare weich und leicht verformbar. Nach einer Zwischenspülung werden die Haare zur Schließung der Disulfidbrücken mit einem Oxidationsmittel behandelt, wobei die gewünschte Formgebung durch Verwendung von beispielsweise Wicklern oder Papilloten erzielt wird.

Wenngleich dieses als Dauerwelle bezeichnete Verfahren heute in großem Umfang angewendet wird, müssen nach wie vor Hilfsmittel eingesetzt werden, die hinsichtlich einer Reihe von Punkten nicht als optimal angesehen werden können.

Als Reduktionsmittel werden heute fast ausschließlich Thioglycolsäure oder deren Salze oder Ester verwendet. Die entsprechenden, alkalisch, d. h. bei einem pH-Wert von ca. 9, eingestellten Zubereitungen zeigen zwar die gewünschte Reduktionsleistung, schädigen jedoch häufig das behandelte Haar, was sich beispielsweise in vermehrt auftretendem Haarbruch äußert. Auch für die Kopfhaut haben diese Mittel vielfach negative Auswirkungen. Es gab daher eine Reihe von Versuchen, verbesserte Reduktionsmittel oder Zubereitungen zu entwickeln. Diese Versuche führten jedoch nicht zu befriedigenden Ergebnissen. So verringert eine saure Einstellung der Zubereitungen zwar die Schäden an Haar und Kopfhaut, jedoch ist die Reduktionswirkung am Haar unzureichend, so daß die Wellungen zu weich sind und die Dauerwelle nur wenig haltbar ist. Die Verwendung anderer Reduktionsmittel führte entweder zu ungenügenden Umformleistungen, oder war mit einer Reihe weiterer Probleme verbunden. Informationen zu dieser Problematik sind beispielsweise dem "Handbuch der Kosmetik und Riechstoffe" von H. Janistyn, Dr. Alfred Hüthig Verlag Heidelberg, 2.Auflage, 1973, in Band III auf den Seiten 353 und folgenden zu entnehmen.

Der Einsatz von Thioglycerin, das gute Umformungsleistungen zeigt, ist beispielsweise toxikologisch bedenklich. Andere, von ihrer Wirksamkeit her ausreichende Verbindungen wie beispielsweise Thioglycolamide werden als sehr instabil gegen Oxidationsmittel bzw. als extrem übelriechend bezeichnet.

Die Verwendung von Cystein und/oder Cystein-Derivaten als alleinige Reduktionsmittel, wie sie beispielsweise in den deutschen Offenlegungsschriften 26 58 424 und 27 17 002 sowie den japanischen Patentanmeldungen 73/14934, 77/125637, 78/101542 und 83/222008 vorgeschlagen wird, ist zwar schonender für Haar und Kopfhaut, führt jedoch häufig nur zu unbefriedigenden Umformleistungen.

Weiterhin wurde in der Europäischen Patentanmeldung 261 387 vorgeschlagen, die Wellwirksamkeit von schwach sauer eingestellten Formulierungen auf Thioglycolsäureester-Basis durch Zugabe von Cysteamin zu erhöhen. Dadurch lassen sich zwar relativ schonende Zubereitungen mit erhöhter Wellaktivität formulieren, jedoch kann die Reduktionswirkung noch immer nicht vollständig befriedigen.

Aus der japanischen Patentanmeldung 57/62217 ist schließlich bekannt, daß die Wirksamkeit von bekannten Reduktionsmitteln für die Dauerwelle wie z.B. Thioglycolsäure, Cystein, Natriumhyposulfit, Natriumsulfit, Thioglycerin und Thiomilchsäure speziell in alkalischem Milieu durch Zugabe von Cysteamin gesteigert werden kann.

Es wurde nun gefunden, daß bei Verwendung von Cysteamin, Cystein und/oder Derivaten des Cysteins mit freier SH-Funktion als Reduktionsmittel in Kombination mit bestimmten weiteren Mercapto-Verbindungen gute Verformungsleistungen bei gleichzeitig außerordentlich geringer Schädigung der Haare möglich sind.

Die Erfindung betrifft daher wäßrige Mittel zur Durchführung der reduzierenden Stufe eines Dauerwellverfahrens für menschliche Haare, dadurch gekennzeichnet, daß sie als Reduktionsmittel mindestens eine Verbindung A, die aus der Gruppe ausgewählt ist, die von Cysteamin, Cystein und den Derivaten des Cysteins mit freier SH-Funktion gebildet wird, in Kombination mit mindestens einer weiteren Mercapto-Verbindung B, ausgewählt aus Mercaptoethanol, Thioäpfelsäure, Thiomilchsäure und α-Mercaptoethansulfonsäure, enthalten.

Diese wäßrigen Mittel werden im weiteren auch als Wellösungen bezeichnet.

Erfindungsgemäß können als Reduktionsmittel (Verbindungen A) Cysteamin, Cystein sowie Derivate des Cysteins mit freier SH-Funktion eingesetzt werden. Geeignete Derivate des Cysteins sind somit beispielsweise N-Acetyl-Cystein, Carbamoyl-Cystein sowie Ester des Cysteins mit Alkoholen mit 1 bis 4 Kohlenstoffatomen. Solche Ester des Cysteins sind beispielsweise der Methyl-, der Ethyl-, der Isopropyl-, der n-Propyl- und der n-Butylester. Cysteamin, Cystein sowie die genannten Cystein-Derivate können sowohl in freier Form als auch in Form von Verbindungen wie beispielsweise den Salzen mit anorganischen oder organischen Säuren eingesetzt werden. Erfindungsgemäß bevorzugt einzusetzende Verbindungen sind die Hydrochloride.

Bevorzugte Verbindungen A sind Cysteamin, Cystein und N-Acetyl-Cystein.

Als weitere Mercapto-Verbindung B wird mindestens eine der Verbindungen Mercaptoethanol, Thioäpfelsäure, Thiomilchsäure oder α-Mercaptoethansulfonsäure eingesetzt. Mercaptoethanol, Thioäpfelsäure und α-Mercaptoethansulfonsäure sind bevorzugte Verbindungen B. Mercaptoethanol ist besonders bevorzugt.

Die als weitere Mercapto-Verbindungen B erfindungsgemäß zu verwendenden Säuren können ebenfalls sowohl als freie Säuren, als auch als Salze, wie beispielsweise als Alkalimetall-, Erdalkalimetallund/oder Ammoniumsalze, eingesetzt werden.

Die genannten Wirkstoffkombinationen zeigen im alkalischen Milieu sehr gute Reduktionswirkungen. Überraschenderweise werden jedoch mit den erfindungsgemäßen Wirkstoffkombinationen besonders im neutralen pH-Bereich, d. h. bei pH-Werten von etwa 6-8, ausgezeichnete Verformungsleistungen beobachtet. Es ist daher möglich, Wellösungen zu formulieren, deren pH-Werte in diesem Bereich liegen. Durch diese Maßnahme wird die Belastung von Haaren und Haut im Vergleich zu alkalisch eingestellten Mitteln entscheidend verringert, so daß die aus diesen Belastungen herrührenden Schädigungen speziell der Haare in weitaus geringerem Umfang auftreten.

Es ist daher erfindungsgemäß bevorzugt, Mittel mit einem pH-Wert von 6-8, insbesondere von 6,5-7,5, zu verwenden.

Die erfindungsgemäßen Mittel enthalten die Verbindungen A bevorzugt in einer Menge von 2-10 Gew.-%, insbesondere in einer Menge von 4-8 Gew.-%, bezogen auf die Gesamtmenge des Mittels.

Die weiteren Mercapto-Verbindungen B werden üblicherweise in gerngeren Mengen als die Verbindungen A eingesetzt. So ist es bevorzugt, diese Verbindungen in Mengen von 0,05-5 Gew.-%, insbesondere in Mengen von 0,1-3 Gew.-%, bezogen auf die Gesamtmenge des Mittels, einzusetzen.

Die erfindungsgemäßen Mittel sind für alle üblichen Zubereitungsformen geeignet. Es ist bevorzugt, die Wellösungen in Form einer Lotion, einer Creme oder eines Gels zu konfektionieren.

Für eine gleichmäßige Wirkung der erfindungsgemäßen Mittel ist eine gleichmäßige Verteilung auf dem Haar notwendig. Dies ist beispielsweise dadurch zu erzielen, daß die Verflüssigungstemperatur der als Mikroemulsion oder thermolabiles Gel eingestellten Wellösung so gewählt wird, daß eine Verflüssigung allein aufgrund der Körperwärme eintritt. Dadurch wird eine Tröpfchenbildung an den Haarspitzen vermieden. Es ist daher bevorzugt, die erfindungsgemäßen Mittel so zu formulieren, daß ihr Verflüssigungspunkt bei einer Temperatur zwischen etwa 20 und 30 °C, und insbesondere bei einer Temperatur von etwa 28 °C, liegt. Der gewünschte Verflüssigungspunkt läßt sich in für den Fachmann bekannter Weise durch die Konzentrationen von konsistenzgebenden Komponenten wie beispielsweise ethoxylierten Fettalkoholen, Polyolfettsäureestern, Fettalkoholen und/oder Paraffinen einstellen. So führt zum Beispiel eine höhere Konzentration an ethoxylierten Fettalkoholen üblicherweise zu höheren Verflüssigungspunkten.

Ebenfalls Gegenstand der Erfindung ist das Verfahren zur Durchführung der reduzierenden Stufe eines Dauerwellverfahrens für menschliche Haare, dadurch gekennzeichnet, daß als Reduktionsmittel mindestens eine Verbindung A, die aus der Gruppe ausgewählt ist, die von Cysteamin, Cystein und den Derivaten des Cysteins mit freier SH-Funktion gebildet wird, in Kombination mit mindestens einer weiteren Mercapto-Verbindung B, ausgewählt aus Mercaptoethanol Thioäpfelsäure, Thiomilchsäure und α-Mercaptoethansulfonsäure, verwendet wird.

Neben der keratinreduzierenden Wirkstoffkombination können die erfindungsgemäßen Wellösungen alle Hilfs- und Zusatzstoffe enthalten, die für die gewünschte Zubereitungsform notwendig oder geeignet sind. Solche Hilfs- und Zusatzstoffe sind in erster Linie oberflächenaktive Stoffe, Konsistenzgeber, Überfettungsmittel, Komplexbildner, haarschonende und haarpflegende Komponenten, Ammoniak und Puffersalze zur Einstellung des gewünschten pH-Wertes, Glycerin, Verdickungsmittel, Duftstoffe, Lösungsvermittler, Farbstoffe und Trübungsmittel.

Als oberflächenaktive Stoffe können Aniontenside, wie beispielsweise Fettalkoholsulfate, Fettalkoholethersulfate, Alkylarylsulfonate oder Sulfosuccinate, Kationtenside, Amphotenside und/oder Niotenside verwendet werden. Es ist bevorzugt, ausschließlich Niotenside, z. B. ethoxylierte Fettalkohole und/oder Aminoxide, oder Amphotenside, wie beispielsweise Fettsäureamid-Derivate mit Betainstruktur, oder Mischungen aus Nio- und Amphotensiden einzusetzen.

Als Konsistenzgeber für Emulsionen können beispielsweise Polyolfettsäureester verwendet werden.

Übliche Überfettungsmittel sind beispielsweise Fettalkohole, Mineralöle und Lanolin.

Bevorzugte Komplexbildner sind Hydroxyethan-1,1-diphosphonsäure, Ethylendiamintetraessigsäure und Nitrilotriessigsäure.

Haarschonende Komponenten sind beispielsweise Lanolinderivate und Polypeptide, aber auch Mittel, die die Quellung des Haares unterstützen wie z. B. Harnstoff und Guanidin.

Der pH-Wert der erfindungsgemäßen Mittel wird in bekannter Weise mit einem Puffersystem eingestellt. Es sind Puffersysteme bevorzugt, die aus Ammoniak und Ammoniumsalzen organischer Säuren, wie beispielsweise Citronensäure, und/oder Ammoniumcarbonat und/oder Ammoniumphosphat bestehen.

Als Verdickungsmittel können beispielsweise Xanthan-Gum, Guarmehl, Cellulosederivate wie Carboxymethylcellulose und Polyacrylate verwendet werden.

Als haarpflegende Komponenten, durch deren Verwendung vor allem sehr glatte Haare erhalten werden, können sowohl kationische als auch anionische Polymere eingesetzt werden. Geeignete kationische Polymere sind beispielsweise quartäre Celluloseether oder Hydroxyalkylcelluloseether, Dimethyl-diallyl-ammoniumchlorid/Acrylamid-Copolymere, oder mit Dialkylsulfat quaternierte Vinylpyrrolidon/Dimethylaminoethylmethacrylat-Copolymere. Verbindungen dieser Art sind beispielsweise unter den Bezeichnungen Polymer JR[R], Celquat[R], Merquat[R] und Gafquat[R] im Handel erhältlich.

Als anionische Polymere können mit Basen vorbehandelte Acrylsäure/Methacrylsäure-Copolymere und/oder Polyacrylate sowie Vinylpyrrolidon/Vinylacetat-Copolymere verwendet werden.

Im Rahmen der erfindungsgemäßen Lehre sind solche Mittel bevorzugt, die ein kationisches Polymer als haarpflegende Komponente enthalten.

Wegen des bereits oben erwähnten, unangenehmen Eigengeruchs der Reduktionsmittel ist die Verwendung von Duftstoffen praktisch unerläßlich. Da viele von ihrer Duftnote her geeignete Parfümöle in den erfindungsgemäßen Mitteln nur unzureichend löslich sind, ist es häufig notwendig, spezielle Lösungsvermittler einzusetzen. Solche Lösungsvermittler sind beispielsweise ethoxylierte, gehärtete Rizinusöle und ethoxylierte Fettalkohole.

Um das optische Erscheinungsbild der erfindungsgemäßen Mittel zu verbessern, können diesen weiterhin Farbstoffe und Trübungsmittel zugesetzt werden.

Übliche Dauerwellverfahren bestehen, wie bereits oben erwähnt, aus einem reduktiven Schritt, einer Zwischenspülung und einem oxidativen Schritt (Fixierung). Gewünschtenfalls können die Haare zusätzlich noch einer Vor- und/oder einer Nachbehandlung unterzogen werden. Die erfindungsgemäßen Mittel können im Rahmen dieser Behandlung mit allen üblichen Fixier-, Vor- und Nachbehandlungsmitteln kombiniert werden. Bestimmte Kombinationen dieser Mittel sind aber bevorzugt.

Die Schädigung der Haare bei Verwendung der erfindungsgemäßen Wirkstoffkombination ist deutlich geringer als bei Verwendung der bekannten Wirkstoffkombinationen. Dieser Effekt ist noch weit größer, wenn der Wellvorgang in Zusammenhang mit einer Blondierung oder oxidativen Färbung der Haare steht. Die erfindungsgemäße Wirkstoffkombination wird daher ebenfalls bevorzugt dann eingesetzt, wenn dem Wellvorgang eine Blondierung oder eine oxidative Färbung der Haare vorausgegangen ist.

Aussehen und Bearbeitbarkeit des zu wellenden Haares kann durch die Verwendung geeigneter Vor- und Nachbehandlungsmittel deutlich verbessert werden.

Vorbehandlungmittel dienen zum Schutz der Haare und vergrößern gewünschtenfalls deren Quellfähigkeit. Solche, in der Regel auf Wasser-Basis formulierte, Mittel enthalten üblicherweise oberflächenaktive Substanzen, kationische oder anionische Polymere, Quellmittel, Parfümöle, Lösungsvermittler für Parfümöle, Stoffe zum Einstellen des gewünschten pH-Wertes, egalisierend wirkende Komponenten wie Sorbit oder Glucose und/oder Farbstoffe.

Nachbehandlungsmittel sollen die Quellung des Haares abbauen, das Haar härten, sprödes Haar weicher erscheinen lassen, die Kämmbarkeit verbessern und die statische Aufladbarkeit des Haares herabsetzen. Entsprechende, wäßrige Mittel enthalten üblicherweise oberflächenaktive Substanzen, anionische oder kationische Polymere, Rückfettungsmittel, die Haarfestigkeit erhöhende Komponenten, wie beispielsweise die in der deutschen Offenlegungsschrift 36 27 746 beschriebenen Wirkstoffkombinationen aus Aluminium(III)-Salzen und Hydroxydi- oder -tricarbonsäuren, vernetzend wirkende Komponenten, Parfümöle und/oder Lösungsvermittler.

Übliche Bestandteile der wäßrigen Fixierlösungen sind Oxidationsmittel, oberflächenaktive Verbindungen, Stabilisatoren, anionische oder kationische Polymere, Substanzen zur Einstellung des, üblicherweise sauren, pH-Wertes, Pflegekomponenten, Verdickungsmittel, Parfümöle und/oder Lösungsmittel.

Vorbehandlungsmittel, Wellösung, Fixierlösung und Nachbehandlungsmittel können unabhängig voneinander sowohl kationisch als auch anionisch formuliert werden. Unter einer kationischen Formulierung soll eine solche Lösung verstanden werden, in der nur kationische Polymere und/oder kationische oberflächenaktive Verbindungen enthalten sind, oder in der diese gegenüber anionischen Polymeren und anionischen oberflächenaktiven Verbindungen deutlich überwiegen. Eine entsprechende Definition gilt für die anionischen Formulierungen.

Es hat sich gezeigt, daß die Naß- und Trockenkämmbarkeit des behandelten Haares verbessert wird, wenn entweder die Wellösung oder die Fixierlösung ein kationisches Polymer enthält und die jeweils andere Lösung anionisch eingestellt ist. Entsprechende Kombinationen sind daher bevorzugt.

Es hat sich als vorteilhaft erwiesen, die einzelnen, aufeinander folgenden Stufen jeweils entgegengesetzt kationisch oder anionisch zu formulieren. Eine kationischen Wellösung wird also bevorzugt zusammen mit einer anionischen Fixierlösung und gegebenenfalls einem anionischen Vorbehandlungsmittel und/oder einem kationischen Nachbehandlungmittel eingesetzt. Gleichfalls bevorzugt ist die Verwendung einer anionischen Wellösung mit einer kationischen Fixierlösung und gegebenenfalls einem kationischen Vorbehandlungsmittel und/oder einem anionischen Nachbehandlungsmittel.

Wellösung, Fixierlösung, Vor- und Nachbehandlungsmittel werden dem Anwender üblicherweise als getrennte Produkte zu Verfügung gestellt. Es ist jedoch auch möglich, die Produkte in einer Mehrkomponentenpackung zu vertreiben, die sowohl Weil- und Fixierlösung, als auch Vor- und/oder Nachbehandlungsmittel enthält.

Gleichfalls ist es möglich, die Wellösung sowohl als einheitliche Lösung zu formulieren als auch in Form eines Wellaktivators, der die Reduktionsmittel enthält, und einer Basislösung aus Pflegemitteln, Substanzen zur pH-Wert-Einstellung, Stabilisatoren und anderen Hilfsmitteln, die erst unmittelbar vor der Anwendung mit dem Wellaktivator vereinigt wird.

Die nachfolgenden Beispiele sollen den Erfindungsgegenstand näher erläutern, ohne ihn jedoch hierauf zu beschränken.

## Beispiele

Die Mengenangaben in den Beispielen sind in allen Fällen Gewichts-%.

## I. Wellösungen

| Beispiel 1: | |
|---|---|
| Cysteaminhydrochlorid | 7,0 |
| Thioäpfelsäure | 2,5 |
| Hydroxyethan-1,1-diphosphonsäure | 0,2 |
| Ammoniak (25%ige Lösung) | ad pH 7, ca. 0,1 |
| Parfümöl | 0,6 |
| gehärtetes Rizinusöl + 60 Ethylenoxid | 4,0 |
| Wasser | ad 100 |

| Beispiel 2: | |
|---|---|
| Cysteaminhydrochlorid | 8,0 |
| α-Mercaptoethansulfonsäure | 1,0 |
| Texapon[R] EVR[1] | 8,0 |
| Hydroxyethan-1,1-diphosphonsäure | 0,2 |
| Ammoniak (25%ige Lösung) | ad pH 7, ca. 0,1 |
| Harnstoff | 5,0 |
| Parfümöl | 0,6 |
| gehärtetes Rizinusöl + 60 Ethylenoxid | 2,0 |
| Wasser | ad 100 |

| Beispiel 3: Wellösung mit Kationpolymer | |
|---|---|
| Cysteaminhydrochlorid | 8,0 |
| Mercaptoethanol | 3,0 |
| Dehyton$^{(R)}$ AB 30[2] | 3,5 |
| Polymer JR$^{(R)}$ 400[3] | 0,2 |
| Hydroxyethan-1,1-diphosphonsäure | 0,2 |
| Ammoniak (25%ige Lösung) | ad pH 7, ca. 0,1 |
| Ammoniumcarbonat | 5,0 |
| Parfümöl | 0,6 |
| Wasser | ad 100 |

| Beispiel 4: Wellösung mit Kationpolymer | |
|---|---|
| Cysteaminhydrochlorid | 7,0 |
| Mercaptoethanol | 2,5 |
| Polymer JR$^{(R)}$ 400[3] | |
| oder Merquat$^{(R)}$ 550[4] | |
| oder Celquat$^{(R)}$ L 200[5] | |
| oder Gafquat$^{(R)}$ 734[6] | 0,3 |
| Hydroxyethan-1,1-diphosphonsäure | 0,2 |
| Ammoniak (25%ige Lösung) | ad pH 7, ca. 0,1 |
| Parfümöl | 0,7 |
| gehärtetes Rizinusöl + 60 Ethylenoxid | 6,0 |
| Wasser | ad 100 |

| Beispiel 5: Wellösung mit Anionpolymer | |
|---|---|
| Cysteaminhydrochlorid | 8,0 |
| α-Mercaptoethansulfonsäure | 3,0 |
| Rohagit S$^{(R)}$[7] | 0,2 |
| Aminoxid WS 35 $^{(R)}$ [8] | 4,0 |
| Ammoniak (25%ige Lösung) | ad pH 7, ca. 0,2 |
| Ammoniumcarbonat | 1,0 |
| Ethylendiamintetraessigsäure | 0,1 |
| Parfümöl | 0,6 |
| Wasser | ad 100 |

| Beispiel 6: Wellösung in Form einer viskosen Mikrodispersion mit einem Verflüssigungspunkt von ca. 29°C | |
|---|---|
| Cysteaminhydrochlorid | 8,0 |
| Mercaptoethanol | 2,0 |
| Cetiol[R] HE[9] | 6,0 |
| Cetylstearylalkohol + 30 Ethylenoxid | 3,0 |
| Decylalkohol | 5,0 |
| Ammoniak (25%ige Lösung) | ad pH 7, ca. 0,1 |
| Ammoniumcarbonat | 1,0 |
| Ethylendiamintetraessigsäure | 0,1 |
| Parfümöl | 0,6 |
| Wasser | ad 100 |

| Beispiel 7: Wellösung in Gelform mit Hydrokolloid | |
|---|---|
| Cysteaminhydrochlorid | 7,0 |
| Mercaptoethanol | 3,0 |
| Dehyton[R] K[10] | 3,0 |
| Kelzan[R] [11] oder Carboxymethylcellulose | 0,4 |
| Ammoniak (25%ige Lösung) | ad pH 7, ca. 0,1 |
| Ammoniumcarbonat | 2,0 |
| Ethylendiamintetraessigsäure | 0,1 |
| Parfümöl | 0,6 |
| Wasser | ad 100 |

| Beispiel 8: Wellösung in Cremeform | |
|---|---|
| Cysteaminhydrochlorid | 8,0 |
| $\alpha$-Mercaptoethansulfonsäure | 2,0 |
| $C_{16}/C_{18}$-Fettalkohol | 16,0 |
| Oleyl/Cetyl-Alkohol + 5 Ethylenoxid | 1,6 |
| Oleyl/Cetyl-Alkohol + 10 Ethylenoxid | 1,0 |
| Eutanol[R] G[12] | 5,0 |
| Ammoniak (25%ige Lösung) | ad pH 7, ca. 0,1 |
| Ammoniumcarbonat | 1,0 |
| Hydroxyethan-1,1-diphosphonsäure | 0,2 |
| Parfümöl | 0,6 |
| Wasser | ad 100 |

| Beispiel 9: Wellgel | |
|---|---|
| Cysteinhydrochlorid | 8,0 |
| Mercaptoethanol | 2,5 |
| Cetiol HE[9] | 6,0 |
| Dodecylalkohol | 5,0 |
| Cetylstearylalkohol + 30 Ethylenoxid | 3,0 |
| Ammoniak (25%ige Lösung) | ad pH 7, ca. 0,1 |
| Ammoniumcarbonat | 1,0 |
| Ethylendiamintetraessigsäure | 0,1 |
| Parfümöl | 0,6 |
| Wasser | ad 100 |

| Beispiel 10: Wellotion | |
|---|---|
| Cystein | 5,0 |
| Cysteamin | 3,0 |
| $\alpha$-Mercaptoethansulfonsäure | 1,5 |
| Hydroxyethan-1,1'-diphosphonsäure | 0,2 |
| Ammoniak (25%ige Lösung) | ad pH 7, ca. 0,1 |
| Harnstoff | 5,0 |
| Dehyton K[10] | 3,0 |
| Parfümöl | 0,6 |
| Wasser | ad 100 |

| Beispiel 11: Wellschaum | |
|---|---|
| Cysteinhydrochlorid | 7,0 |
| Mercaptoethanol | 3,0 |
| Ethylendiamintetraessigsäure | 0,1 |
| Nonylphenolpoly(7 EO)glykolether | 3,0 |
| Natriumlauryl(3 EO)ethersulfat | 2,0 |
| Ammoniak (25%ige Lösung) | ad pH 7, ca. 0,1 |
| Parfümöl | 0,6 |
| Wasser | ad 100 |

| Beispiel 12: Wellotion mit kationischem Polymer | |
|---|---|
| N-Acetyl-Cystein | 8,0 |
| Mercaptoethanol | 2,0 |
| Ethylendiamintetraessigsäure | 0,1 |
| Gafquat[R] 734[6] | 0,3 |
| Lamepon[R] S[23] | 3,0 |
| Ammoniak (25%ige Lösung) | ad pH 7,3, ca. 0,3 |
| Ammoniumcarbonat | 2,0 |
| Parfümöl | 0,6 |
| Wasser | ad 100 |

| Beispiel 13: Wellotion mit anionischem Polymer | |
|---|---|
| N-Acetyl-Cystein | 4,0 |
| Cysteamin | 4,0 |
| Mercaptoethanol | 2,0 |
| Ethylendiamintetraessigsäure | 0,1 |
| Stearylpoly(4 EO)ethylenglykolcarboxylat-Natriumsalz | 4,0 |
| Ammoniak (25%ige Lösung) | ad pH 7, ca. 0,3 |
| Ammoniumcarbonat | 3,0 |
| Rohagit S$^{(R)7}$ | 0,2 |
| Parfümöl | 0,6 |
| Wasser | ad 100 |

| Beispiel 14: Wellcreme | |
|---|---|
| Cystein | 8,0 |
| Mercaptoethanol | 3,0 |
| Ethylendiamintetraessigsäure | 0,1 |
| Cetyl/Stearylalkohol | 4,0 |
| Cetyl/Stearylsulfat | 2,0 |
| gehärtetes Rizinusöl + 60 Ethylenoxid | 3,5 |
| Ammoniak (25%ige Lösung) | ad pH 7,4, ca. 0,4 |
| Ammoniumcarbonat | 3,0 |
| Parfümöl | 0,6 |
| Wasser | ad 100 |

## II. Fixiermittel

| Beispiel 15: kationisch eingestellte Fixierlösung (bevorzugt verwendet mit einer anionisch eingestellten erfindungsgemäßen Wellösung) | |
|---|---|
| Dehyton$^{(R)}$ G$^{13}$ | 4,0 |
| Polymer JR$^{(R)}$ 400$^3$ | 0,3 |
| Phosphorsäure | 0,5 |
| Wasserstoffperoxid | 2,0 |
| Hydroxyethan-1,1-diphosphonsäure | 0,5 |
| Proteinhydrolysat (Kollagen oder Keratin) | 0,8 |
| Parfümöl | 0,5 |
| Wasser | ad 100 |

9

| Beispiel 16: anionisch eingestellte Fixierlösung (bevorzugt verwendet mit einer kationisch eingestellten erfindungsgemäßen Wellösung) | |
|---|---|
| Texapon[R] N 25[14] | 3,0 |
| Phosphorsäure | 0,5 |
| Wasserstoffperoxid | 2,0 |
| Hydroxyethan-1,1-diphosphonsäure | 0,5 |
| Proteinhydrolysat (Kollagen oder Keratin) | 0,8 |
| Parfümöl | 0,5 |
| gehärtetes Rizinusöl + 60 Ethylenoxid | 3,0 |
| Wasser | ad 100 |

| Beispiel 17: anionisch eingestellte Fixierlösung (bevorzugt verwendet mit einer kationisch eingestellten erfindungsgemäßen Wellösung) | |
|---|---|
| Carbopol 940[R] [15] | 0,2 |
| Aminol[R] A 15[16] | 2,0 |
| Phosphorsäure | 0,5 |
| Natriumbromat | 8,0 |
| Carrageen[17] | 0,1 |
| Avocado-Öl | 0,2 |
| Parfümöl | 0,8 |
| gehärtetes Rizinusöl + 60 Ethylenoxid | 6,0 |
| Wasser | ad 100 |

### III. Vorbehandlungsmittel

| Beispiel 18: kationisch eingestelltes Vorbehandlungsmittel (bevorzugt verwendet mit einer anionisch eingestellten erfindungsgemäßen Wellösung) | |
|---|---|
| Sorbit | 0,4 |
| Glucose | 1,0 |
| Polymer JR[R] 400[3] | 0,2 |
| Ammoniumcarbonat | 1,0 |
| Parfümöl | 0,2 |
| gehärtetes Rizinusöl + 60 Ethylenoxid | 3,0 |
| Wasser | ad 100 |

| Beispiel 19: anionisch eingestelltes Vorbehandlungsmittel (bevorzugt verwendet mit einer kationisch eingestellten erfindungsgemäßen Wellösung) | |
|---|---|
| Harnstoff | 2,0 |
| Carbopol 940[R] [15] | 0,2 |
| Texapon[R] N 25[14] | 2,0 |
| Glucam[R] P 20[18] | 1,0 |
| Farbstoff | 0,02 |
| Wasser | ad 100 |

## IV. Nachbehandlungsmittel

| Beispiel 20: kationisch eingestelltes Nachbehandlungsmittel (bevorzugt verwendet mit einer kationisch eingestellten erfindungsgemäßen Wellösung und einer anionisch eingestellten Fixierlösung) | |
|---|---|
| Gafquat[R] 755[19] | 0,3 |
| Citronensäure | 0,5 |
| Gluconsäure | 0,2 |
| Magnesiumchlorid | 2,0 |
| Parfümöl | 0,3 |
| gehärtetes Rizinusöl + 40 Ethylenoxid | 4,0 |
| Wasser | ad 100 |

| Beispiel 21: anionisch eingestelltes Nachbehandlungsmittel (bevorzugt verwendet mit einer anionisch eingestellten erfindungsgemäßen Wellösung und einer kationisch eingestellten Fixierlösung) | |
|---|---|
| Luviskol[R] VA 64[20] oder Carbopol[R] 940[15] | 0,2 |
| Texapon[R] N 70 [21] | 2,0 |
| Äpelsäure | 0,4 |
| Aluminium(III)-lactat | 2,0 |
| $C_{16}/C_{18}$-Fettalkohol | 1,0 |
| Oleyl/Cetyl-Alkohol + 10 Ethylenoxid | 3,0 |
| DC[R] 190[22] | 0,2 |
| Parfümöl | 0,5 |
| Wasser | ad 100 |

[1] Texapon[R] EVR: Natriumlaurylethersulfat, ca.36% Aktivsubstanz in Wasser (Fa. Henkel)

[2] Dehyton[R] AB 30: Fettamin-Derivat mit Betainstruktur, ca. 30% Aktivsubstanz in Wasser, CTFA-Bezeichnung: Coco-Betaine (Fa. Henkel)

[3] Polymer JR 400[R]: quaternierte Hydroxyethylcellulose (Fa. Union Carbide)

[4] Merquat[R] 550: Dimethyldiallylammoniumchlorid/Acrylamid-Copolymer (Fa. Merck)

[5] Celquat[R] L 200: quaterniertes Cellulose-Derivat (Fa. Delft National)

[6] Gafquat[R] 734: quaternäres Vinylpyrrolidon-Dimethylaminomethacylat-Copolymer, mit Diethylsulfat quaterniert, ca. 50 % im Ethanol (Fa. GAF)

[7] Rohagit S[R]: Mit Base umgesetzte Polymethacrylsäure (Fa. Röhm)

[8] Aminoxid WS 35 [R]: Dimethylkokosacylamidopropylaminoxid (Fa. Goldschmidt)

[9] Cetiol HE[R]: Polyolfettsäureester (Fa. Henkel)

[10] Dehyton K[R]: Fettsäureamid-Derivat mit Betainstruktur der Formel $R-CONH-(CH_2)_3-N^+(CH_3)_2-CH_2-COO^-$, ca. 30% Aktivsubstanz in Wasser (Fa. Henkel)

[11] Kelzan [R]: Xanthan-Gum (Fa. Kelco)

[12] Eutanol[R] G: Langkettige, verzweigte Alkohole (Fa. Henkel)

[13] Dehyton[R] G: N-Hydroxyethyl-N-kokosalkylamidoethyl-glycinat, Natrium-Salz, ca. 30% Aktivsubstanz in Wasser (Fa. Henkel)

[14] Texapon[R] N 25: Natriumlaurylethersulfat, ca. 28 % Aktivsubstanz in Wasser (Fa. Henkel)

[15] Carbopol 940[R]: Polyacrylsäure (Fa. Goodrich)

[16] Aminol[R] A 15: Laurylalkohol + 2 Ethylenoxid-essigsäuremonoethanolamid (Fa. Chem-Y)

[17] Carrageen: $\beta$-D-Galactopyranose-2-sulfat (Fa. Ceca)

[18] Glucam[R] P 20: Methylglucosid + 20 Propylenoxid (Fa. Amerchol)

[19] Gafquat[R] 755: Dimethylaminoethylmethacrylat-Vinylpyrrolidon-Copolymer, mit Diethylsulfat quaterniert, 20% in Wasser (Fa. GAF)

[20] Luviskol[R] VA 64: Vinylacetat/Vinylpyrrolidon-Copolymer, 40:60 (Fa. BASF)

[21] Texapon[R] H 70: Natriumlaurylethersulfat, ca. 70% Aktivsubstanz in Wasser (Fa. Henkel)

[22] DC[R] 190: Siliconglycol-Copolymer (Fa. Dow Corning)

[23] Lamepon[R]S: Eiweißhydrolysat-Fettsäure-Kondensat-Kaliumsalz (Fa. Grünau)

## V. Anwendungstests

Die Schädigung der Haare wurde nach dem im folgenden geschilderten Verfahren bestimmt.

Haarsträhnen mit einem Gewicht von ca. 2000 mg wurden genau gewogen und anschließend abwechselnd je fünfmal mit einem handelsüblichen Blondiermittel mit einem Gehalt von 12% $H_2O_2$ und der zu prüfenden Wellösung behandelt. Nach dieser extremen Haarbelastung wurde das Haar konditioniert und vorsichtig durchgekämmt. Das verbleibende Haar wurde nun erneut gewogen. Der auftretende Haarbruch ist ein Maß für die Haarschädigung.

Die Haarschädigung S wurde definiert zu

$$S = \frac{B-A}{B} \times 100\%$$

mit A = Gewicht der behandelten Haare,

B = Gewicht der Haare vor der Behandlung.

Bei Verwendung üblicher Wellösungen mit hoher Welleistung wurden Schädigungen in der Größenordnung von 40 - 50% beobachtet. Bei Anwendung der erfindungsgemäßen Wellösungen wurden dagegen nur noch Schädigungen von ca. 10 - 20% beobachtet.

Die Welleistungen der verwendeten Wellösungen wurden durch Versuche am Wellbrett nach Kirby ermittelt. Einzelheiten zu diesem Testverfahren sind den Veröffentlichungen von J. Kirby in Drug Cosmet. Ind. 80, S. 314 (1957) und Kosmetik-Parfüm-Drogen RDSCH (Innsbruck) 5, S. 3 (1958) zu entnehmen.

Übliche, alkalisch formulierte Wellmittel mit Thioglykolsäure als Reduktionsmittel zeigten Wellwerte von ca. 8,5 cm, bekannte, neutral oder sauer eingestellte Wellmittel dagegen nur Werte von ca. 10,5-11 cm.

Bei Verwendung der erfindungsgemäßen Wellmittel wurden Wellwerte von ca. 8,5 cm gefunden. Die erfindungsgemäßen Mittel zeigen somit eine Welleistung, die der bekannter Produkte mit hoher Welleistung entspricht.

## Ansprüche

1. Wäßrige Mittel zur Durchführung der reduzierenden Stufe eines Dauerwellverfahrens für menschliche Haare, dadurch gekennzeichnet, daß sie als Reduktionsmittel mindestens eine Verbindung A, die aus der Gruppe ausgewählt ist, die von Cysteamin, Cystein und den Derivaten des Cysteins mit freier SH-Funktion gebildet wird, in Kombination mit mindestens einer weiteren Mercapto-Verbindung B, ausgewählt aus Mercaptoethanol, Thioäpfelsäure, Thiomilchsäure und α-Mercaptoethansulfonsäure, enthalten.

2. Mittel nach Anspruch 1, dadurch gekennzeichnet, daß sie als Verbindung A Cysteamin enthalten.

3. Mittel nach Anspruch 1, dadurch gekennzeichnet, daß sie als Verbindung A Cystein und/oder N-Acetyl-Cystein enthalten

4. Mittel nach einem der Ansprüche 1 bis 3, dadurch gekennzeichnet, daß sie als weitere Mercapto-Verbindung B Mercaptoethanol, Thioäpfelsäure und/oder α-Mercaptoethansulfonsäure enthalten.

5. Mittel nach einem der Ansprüche 1 bis 4, dadurch gekennzeichnet, daß sie als weitere Mercapto-Verbindung B Mercaptoethanol enthalten.

6. Mittel nach einem der Ansprüche 1 bis 5, dadurch gekennzeichnet, daß sie einen pH-Wert von 6-8, insbesondere von 6,5-7,5 aufweisen.

7. Mittel nach einem der Ansprüche 1 bis 6, dadurch gekennzeichnet, daß sie 2-10 Gew.-%, insbesondere 4-8 Gew.-%, Verbindungen A und 0,05-5 Gew.-%, insbesondere 0,1-3 Gew.-%, an weiteren Mercapto-Verbindungen B enthalten.

8. Mittel nach einem der Ansprüche 1 bis 7, dadurch gekennzeichnet, daß sie als Lotion, Gel oder Creme vorliegen.

9. Verfahren zur Durchführung der reduzierenden Stufe eines Dauerwellverfahrens für menschliche Haare, dadurch gekennzeichnet, daß als Reduktionsmittel mindestens eine Verbindung A, die aus der Gruppe ausgewählt ist, die von Cysteamin, Cystein und den Derivaten des Cysteins mit freier SH-Funktion gebildet wird, in Kombination mit mindestens einer weiteren Mercapto-Verbindung B, ausgewählt aus Mercaptoethanol, Thioäpfelsäure, Thiomilchsäure und α-Mercaptoethansulfonsäure, verwendet wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-1 003 963 (K. SCHWARZKOPF) <br> * Ansprüche; Beispiel 7 * <br> --- | 1,3,4,6 -9 | A 61 K 7/09 |
| Y | EP-A-0 095 916 (PROCTER & GAMBLE) <br> * Ansprüche * <br> --- | 1-9 | |
| D,Y | EP-A-0 261 387 (WELLA) <br> * Ansprüche; Beipiel 1 * <br> ----- | 1-9 | |

RECHERCHIERTE SACHGEBIETE (Int. Cl.5)

A 61 K

Der vorliegende Recherchenbericht wurde für alle Patentansprüche erstellt

| Recherchenort | Abschlußdatum der Recherche | Prüfer |
|---|---|---|
| DEN HAAG | 20-11-1989 | WILLEKENS G.E.J. |